# EUROPEAN PATENT APPLICATION

(11) **EP 4 079 850 A1**
(43) Date of publication of application: **26.10.2022**
(21) Application number: 20900777.2
(22) Date of filing: 11.12.2020
(51) Int. Cl.: C12N 15/09, C12Q 1/68

(54) **ANALYTICAL METHOD AND KIT**

(30) Priority: 17.12.2019 WO PCT/JP2019/049450
(71) Applicant: KABUSHIKI KAISHA TOSHIBA, Minato-ku Tokyo 105-0023 (JP)
(72) Inventor: INADA, Mika, Tokyo 105-0023 (JP); HASHIMOTO, Koji, Tokyo 105-0023 (JP)
(74) Representative: Marks & Clerk LLP
(86) International application number: PCT/JP2020/046391
(87) International publication number: WO 2021/125105

(57) **Abstract**

According to one embodiment, there is provided an analytical method for determining the presence or absence of contraction of cancer in a subject, which includes quantifying hsa-miR-1301-3p in a sample originating from the subject.

## Description

### Technical Field

The embodiments of the present invention relate to an analytical method and a kit.

### Background Art

In recent years, attention has been paid to the relationship between microRNA (miRNA) and diseases. It has been reported that miRNAs have a function of regulating gene expression, and the types and expression levels of miRNAs undergo changes from an early stage in various diseases. That is, in patients with a certain disease, the amount of a specific miRNA is increased or decreased as compared with healthy subjects. Therefore, examining the amount of the miRNA in a sample collected from an examinee is a means of knowing whether the patient has the disease or not.

### Summary of Invention

### Technical Problem

The present invention aims to provide an analytical method, a kit, a primer set and a detection device that can conveniently determine the presence or absence of contraction of cancer in a subject.

### Solution to Problem

According to embodiments, there is provided an analytical method for determining the presence or absence of contraction of cancer in a subject, the method including quantifying hsa-miR-1301-3p in a sample originating from the subject.

### Brief Description of Drawings

FIG. 1 is a flow chart showing an example of an analytical method of a first embodiment.
FIG. 2 is a flow chart showing an example of an analytical method of a second embodiment.
FIG. 3 is a flow chart showing an example of a quantification step of a third embodiment.
FIG. 4 is a schematic diagram showing an example of a quantification step of the third embodiment.
FIG. 5 is a box-and-whisker plot showing the experimental results of Example 1.
FIG. 6 is a box-and-whisker plot showing the experimental results of Example 1.
FIG. 7 is a box-and-whisker plot showing the experimental results in the case of using primer set A of Example 2.
FIG. 8 is a box-and-whisker plot showing the experimental results of Example 2.
FIG. 9 is a box-and-whisker plot showing the experimental results of Example 2.
FIG. 10 is a box-and-whisker plot showing the experimental results in the case of using primer set J of Example 2.
FIG. 11 is a box-and-whisker plot showing the experimental results of Example 2.
FIG. 12 is a box-and-whisker plot showing the experimental results of Example 2.

### Mode for Carrying Out the Invention

In the following description, the analytical method and the kit of the embodiments will be described with reference to the drawings.

### • First embodiment

### (Analytical method)

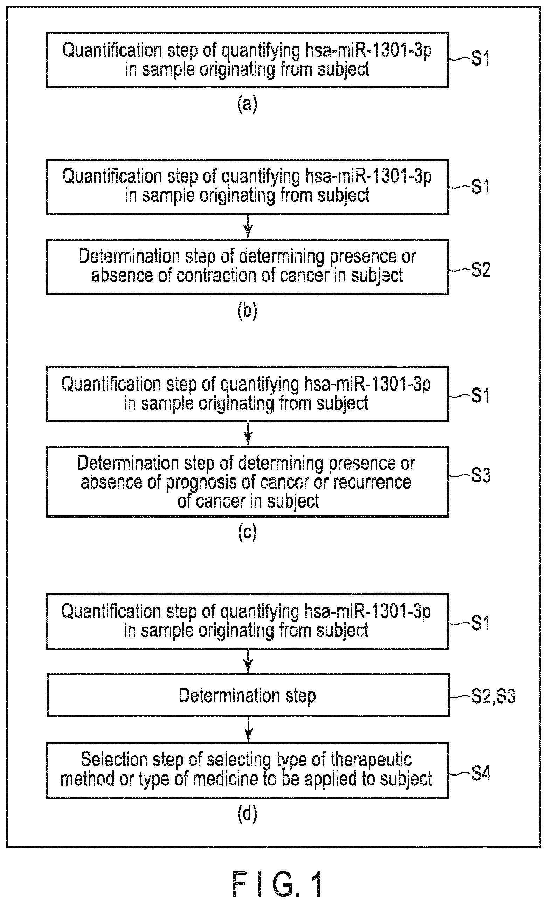

As shown in part (a) of FIG. 1, an analytical method according to a first embodiment is a method for determining the presence or absence of contraction of cancer in a subject, the method including quantifying hsa-miR-1301-3p in a sample originating from the subject (quantification step (S1)).

Cancer refers to a malignant tumor, a malignant neoplasm, a cancer, and a sarcoma. Cancer is not limited; however, examples include prostate cancer, breast cancer, colorectal cancer, stomach cancer, lung cancer, ovarian cancer, pancreatic cancer, bile duct cancer, esophageal cancer, liver cancer, brain tumor, urinary bladder cancer, sarcoma, uterine body cancer, uterine sarcoma, mesothelioma, pharyngeal cancer, maxillary cancer, oral cancer, tongue cancer, lip cancer, laryngeal cancer, thyroid cancer, nerve tumor, glioma, neuroblastoma, renal cell cancer, testis cancer, cervical cancer, skin cancer, malignant melanoma, bone tumor, leukemia, malignant lymphoma, and multiple myeloma.

Cancer also includes cancers of all stages of disease, and examples include a condition in which cancer has stayed in an organ of the origin of tumor, a condition in which cancer has further spread to surrounding tissues, a condition in which cancer has further metastasized to lymph nodes, and a condition in which cancer has metastasized to a more distant organ.

The subject is an animal that is subjected to analysis in the present method, that is, an animal that provides a sample. The subject may be an animal having any disease or may be a healthy animal. For example, the subject may be an animal having a possibility of contracting cancer, an animal that was affected with cancer in the past, or the like.

The subject is preferably a human being. Alternatively, the subject may also be another animal. The other animal is, for example, a mammal, and examples include animals belonging to primates such as monkey; rodents such as mouse, rat, and guinea pig; companion animals such as dog, cat, and rabbit; domestic animals such as horse, cattle, and pig; and display animals.

A sample originating from a subject includes a sample collected from the subject, a sample obtained by appropriately treating the above-mentioned sample, and the like. The sample is preferably blood serum. The sample may also be other body fluids, for example, blood, blood plasma, white blood cells, interstitial fluid, urine, feces, sweat, saliva, oral mucosa, intranasal mucosa, nasal mucus, pharyngeal mucosa, sputum, digestive juices, gastric juice, lymph, cerebrospinal fluid, tear fluid, breast milk, amniotic fluid, semen, or vaginal fluid. Alternatively, the sample may be a tissue, cells, or the like and may also be a tissue or cells collected from the subject and cultured, or a supernatant thereof.

In the following description, an example of the procedure of the analytical method of the first embodiment will be described.

First, a sample originating from a subject is prepared. The sample can be collected using any common method according to the type of the sample. The sample may be used as it is after collection or may be treated so as to be in a state that does not inhibit a reaction for quantification of nucleic acid or a state that is more suitable for the reaction. Examples of the treatment include shredding, homogenization, centrifugation, precipitation, extraction, and/or separation, and the treatment can be carried out by any known means.

For example, extraction may be performed using a commercially available nucleic acid extraction kit. As the nucleic acid extraction kit, for example, NucleoSpin (registered trademark) miRNA Plasma (manufactured by Takara Bio, Inc.), Quick-cfRNA Serum & Plasma Kit (manufactured by Zymo Research Corp.), miRNeasy Serum/Plasma Kit (manufactured by Qiagen GmbH), miRVana PARIS isolation kit (manufactured by Thermo Fisher Scientific, Inc.), PureLinkTM Total RNA Blood Kit (manufactured by Thermo Fisher Scientific, Inc.), Plasma/Serum RNA Purification Kit (manufactured by Norgen Biotech Corp.), microRNA Extractor (registered trademark) SP Kit (Wako Pure Chemical Industries, Ltd.), and High Pure miRNA Isolation Kit (manufactured by Sigma-Aldrich Corp.) can be used. Alternatively, extraction may also be performed, without using a commercially available kit, for example, by diluting the sample with a buffer solution, performing a heating treatment at 80°C to 100°C, centrifuging the sample, and collecting the supernatant thereof.

Next, quantification of hsa-miR-1301-3p in the sample is performed (quantification step (S1)). hsa-miR-1301-3p is an miRNA having the following sequence: UUGCAGCUGCCUGGGAGUGACUUC (SEQ ID NO: 1).

Hereinafter, hsa-miR-1301-3p is also referred to as "target miRNA".

A quantification step (S1) can be carried out using a general method for quantifying an RNA, particularly a short stranded RNA such as miRNA. The method is not limited; however, for example, it is preferable that the target miRNA is subjected to reverse transcription, a cDNA is amplified, and an amplification product is detected and quantified. Regarding amplification, for example, a PCR method, a qPCR method, a LAMP method, and the like can be used. Detection and quantification may be performed after amplification or may be performed over time during amplification. Regarding the detection and quantification, for example, a measurement method of using a signal based on turbidity or light absorbance, a measurement method of using an optical signal, a measurement method of using an electrochemical signal, or combinations of these can be used. For example, quantification of a target miRNA can be carried out from detection results such as the intensity or the amount of change of the above-described signal obtainable in accordance with the amount of the amplification product, the time for the signal to reach a threshold value (rise time), or the number of cycles (number of rising cycles) in the case of using a PCR method.

A quantitative value of the target miRNA can be determined using a calibration curve showing the relationship between the detection results of the signal and the abundance of the target miRNA. The calibration curve can be produced by performing detection of signals for a plurality of standard samples containing the target miRNA at different known concentrations. By comparing this calibration curve with the detection result of the signal obtained for a sample originating from the subject, an abundance of the target miRNA in the sample can be calculated. For example, the abundance of the target miRNA in a sample can be obtained as a number of copies of the target miRNA per unit amount of the sample.

The quantification step (S1) may be carried out using, for example, a commercially available kit. Examples of the commercially available kit include TaqMan (registered trademark) Advanced miRNA Assays (manufactured by Thermo Fisher Scientific, Inc., ID: 477827_mir), miRCURY LNA miRNA PCR Assays (manufactured by Qiagen Corp., catalogue No. YP02103132), and SYBR (registered trademark) Green qPCR microRNA detection system (manufactured by Origin Technologies Group, Inc.), and the kits can be used together with primers that specifically amplify the target miRNA.

A more preferred example of the quantification step (S1) will be described in a third embodiment.

A quantitative value of the target miRNA obtained in the quantification step (S1) can be used in order to determine the presence or absence of contraction of cancer in a subject.

For example, the analytical method of the first embodiment can further include a determination step (S2) that can be carried out after the quantification step (S1), as shown in part (b) of FIG. 1. In the determination step (S2), when the quantitative value of the target miRNA is high, it is determined that the subject has contracted cancer. In contrast, when the quantitatively value is low, it is determined that the subject is not affected with cancer. The level of the quantitative value is determined based on, for example, the quantitative value or the threshold value of the target miRNA in a control obtained in advance. For example, when the quantitative value is higher than those, the subject can be determined to have contracted cancer.

The control is, for example, a normal subject. The normal subject refers to an individual that is at least not affected with cancer. The normal subject is preferably a healthy individual that does not have any disease or abnormality. An individual selected as a control may be an individual that is different from the subject to be analyzed by the present method; however, the control is preferably an individual belonging to the same species, that is, when the subject is a human being, the control is preferably a human being. Furthermore, the age, gender, and physical conditions such as height and body weight of the control or the number of persons are not particularly limited; however, it is preferable that the physical conditions are the same as or similar to those of the subject to be examined by the present analytical method.

The quantitative value of the target miRNA in the control is, for example, a quantitative value of the target miRNA obtained by using a sample that is the same as or similar to that originating from the control and using the same method as that used in the quantification step (S1).

The threshold value is, for example, a quantitative value of the target miRNA, by which the quantitative values for the subject that has contracted cancer and the quantitative values for the control that is not affected with cancer, can be divided. The threshold value can be determined, for example, by using the same or similar sample originating from a standard subject, for which it is known whether the standard subject has contracted cancer, or a sample containing an established cell line of a cancer, from a quantitative value of the target miRNA obtained by a method similar to that used in the quantification step (S1). The threshold value is not limited and can be determined according to the quantification method, the type of the sample, measurement conditions, which are to be used and the like.

Alternatively, the threshold value may be determined for each subject. For example, in a case where the quantitative value of the target miRNA of a subject in a healthy condition, the quantitative value being regularly measured by medical examination or the like, has been monitored, when the quantitative value in a healthy condition is used as the threshold value, and when a quantitative value is found to be higher than the threshold value, it can be determined that there is a possibility of contracting cancer, and an alarm can be given out. The threshold value can vary from an individual to an individual. For example, for a subject A in which the quantitative value of the target miRNA usually changes at about 10³ copies, when the quantitative value reaches 10⁴ copies at one point, the subject can be considered to have a possibility of cancer. On the other hand, for a subject B in which the quantitative value changes at about 10² copies, when the quantitative value reaches 10³ copies at one point, the subject can be considered to have a possibility of cancer.

The quantitative value or the threshold value for the control may be determined from findings in the past, such as literature.

Here, contracting a disease also includes having a high possibility of contracting a disease. On the contrary, not being affected with a disease also includes having a low possibility of contracting a disease.

According to another embodiment, determining that a subject has contracted cancer also includes determining the prognosis or recurrence of cancer in the subject. For example, as shown in part (c) of FIG. 1, the analytical method includes, after the quantification step (S1), a determination step (S3) of determining the presence or absence of the prognosis or recurrence of cancer in a subject from the results of quantification. In the determination step (S3), for example, when the quantitative value is high, it can be determined that the prognosis of cancer in the subject is bad or that cancer has recurred, or the possibility of recurrence is high. The quantitative value and the threshold value for the control can also be used in the determination of prognosis and recurrence. In particular, it may be preferable to use a threshold value determined for each subject.

Furthermore, after the determination step (S2) and/or the determination step (S3), it is possible to select the type of a therapeutic method or the type of a medicine to be applied to the subject according to the determination results. For example, as shown in part (d) of FIG. 1, the analytical method includes, after the determination step (S2) and/or the determination step (S3), a selection step (S4) of selecting the type of the therapeutic method or the type of the medicine to be applied to the subject from the determination results. Here, the therapeutic method or the medicine is a therapeutic method or medicine for cancer treatment. The type of the therapeutic method or the type of the medicine includes a quantity of use, timing, or duration of the therapeutic method or the medicine.

According to the analytical method of the first embodiment described above, it is possible to conveniently determine the presence or absence of the contraction of cancer in a subject, by quantifying one kind of target miRNA (hsa-miR-1301-3p). In other words, according to the present method, a subject that has contracted cancer and a subject that is not affected with cancer can be easily distinguished.

Particularly, since the analytical method of the embodiments can use blood serum that can be easily collected by medical examination or the like, cancer can be detected in early stages. When blood serum or the like is used, the physical and economic burden on the subject can be significantly reduced as compared with cytodiagnosis and the like, and in addition, since the procedure is simple, the burden on the examiner is small. Furthermore, regarding the blood serum, since the concentration of the miRNA included therein is stabilized, more accurate tests can be performed.

According to still another embodiment, there is also provided an analytical method for assisting in determining the presence or absence of contraction of cancer in a subject, the analytical method including quantifying a target miRNA in a sample originating from the subject (quantification step (S1)).

The term "assisting in determining" includes, for example, acquiring information on a possibility that a subject may have contracted cancer. The "information" may be, for example, a quantitative value obtained in the quantification step (S1). According to the present method, more accurate information for implementing determining the presence or absence of contraction of cancer in a subject, determining the prognosis, determining the presence or absence of recurrence, selecting a therapeutic method or a medicine to be applied to the subject, or the like can be acquired.

According to still another embodiment, the present analytical method can also be used for detecting cancer cells in a sample that is not originated from a subject. For example, the present analytical method can also be used in a case where cancer cells are artificially produced, and at the time of checking whether cancer cells are present in a solution containing the produced cells or not.

### (Marker)

According to the first embodiment, there is provided a marker for detecting cancer, the marker including hsa-miR-1301-3p.

Here, the term "marker" refers to a substance that allows determining whether a sample and/or a subject from which the sample originates is in a specific condition, by detecting the presence or absence of the substance or the concentration of the substance in the sample.

The marker for cancer detection of the first embodiment allows, for example, implementation of determining the presence or absence of contraction of cancer in a subject, determining the prognosis, determining the presence or absence of recurrence, selecting a therapeutic method or a medicine to be applied to the subject, or the like as described above, by measuring the abundance (quantitative value) of the marker in a sample originating from the subject.

### (Kit)

According to the first embodiment, a kit for cancer detection is provided.

This kit includes at least one nucleic acid selected from the group consisting of a reverse transcription (RT) primer for reverse transcribing hsa-miR-1301-3p, an elongation (EL) primer for elongating hsa-miR-1301-3p, an amplification primer set for amplifying hsa-miR-1301-3p, and a nucleic acid probe for detecting hsa-miR-1301-3p.

An RT primer is a primer for obtaining cDNA of a target miRNA. The RT primer includes a sequence complementary to the sequence of at least a part of the target miRNA. The RT primer may also include an artificial sequence to be added to the cDNA, which facilitates amplification of the cDNA of the target miRNA.

An EL primer is a primer for adding an artificial sequence to the cDNA in order to facilitate amplification of the cDNA of a target miRNA. The EL primer can include a sequence complementary to the sequence of at least a part of the cDNA of the target miRNA, and a sequence to be added for elongation of the cDNA.

An amplification primer set is, for example, intended for a PCR method and includes at least a forward primer and a reverse primer. Alternatively, the amplification primer set is intended for a LAMP method and includes at least an FIP primer and a BIP primer. The amplification primer set may also include an F3 primer, a B3 primer, and/or a loop primer, as necessary.

Each of the primers included in the amplification primer set may be designed to bind to the cDNA of the target miRNA or a complementary sequence thereof, or may be designed to bind to an artificial sequence added by an RT primer and/or an EL primer. Preferred sequences of the RT primer, the EL primer, and the amplification primer set will be described in a third embodiment.

A nucleic acid probe can have the sequence of at least a part of the target miRNA, the cDNA thereof, or an amplification product thereof, or a complementary sequence of the sequence.

The nucleic acids to be included in the kit may be provided individually or in combination in the form of being accommodated in a container together with an appropriate carrier. The appropriate carrier is, for example, water, a physiological solution, or a buffer solution. The container is, for example, a tube or a microtiter plate. Alternatively, these nucleic acids may be provided in the form of being immobilized on a solid phase of a microfluid chip or the like.

The kit may also include, in addition to the above-described nucleic acids, a reagent used for reverse transcription, elongation, and/or amplification, for example, an enzyme, a substrate, and/or a labeling substance generating an optical signal or an electrochemical signal to be used for detection. Examples of the labeling substance include a fluorescent dye such as SYBR Green, EVA green, or SYTO 82; and in the case of detecting an electric current, an indicator such as a metal complex such as ruthenium hexaamine.

The kit can be used, for example, in determining the presence or absence of contraction of cancer in a subject, determining the prognosis, determining the presence or absence of recurrence, and selecting the type of a therapeutic method or the type of a medicine, as described above.

According to another embodiment, the kit for cancer detection is provided as a diagnostic composition or a diagnostic agent for cancer. Furthermore, according to the embodiments, there is also provided the use of at least one of the above-described nucleic acids for the production of a diagnostic composition for cancer or a diagnostic agent for cancer.

### • Second embodiment

### (Analytical method)

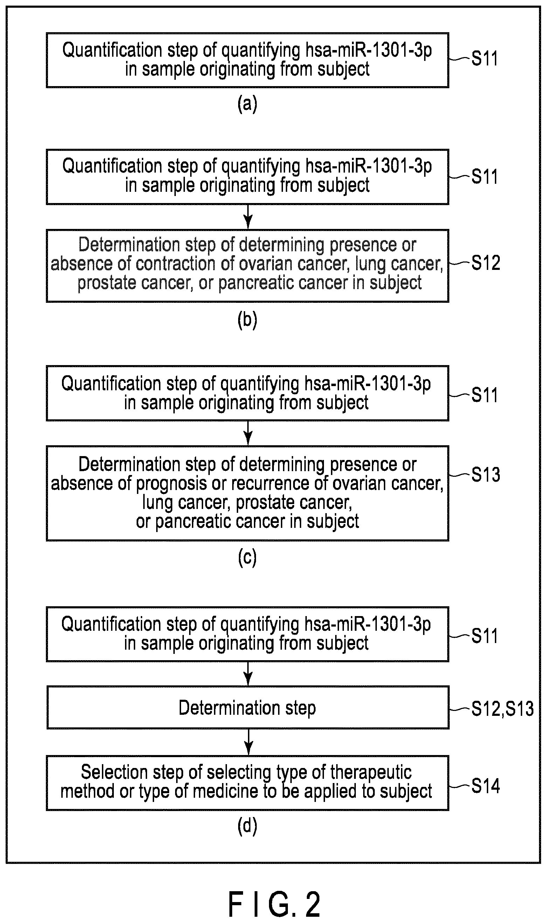

An analytical method of a second embodiment is, as shown in part (a) of FIG. 2, a method for determining the presence or absence of contraction of at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, and uterine body cancer in a subject, the method including quantifying hsa-miR-1301-3p in a sample originating from the subject (quantification step (S11)).

Breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, and uterine body cancer will be collectively referred to as "target cancers" in the following description.

The quantification step (S11) can be carried out similarly to the quantification step (S1) of the first embodiment; however, amplification can be carried out using a LAMP method. The subject and the sample can be similar to those of the first embodiment; however, the subject may be an animal having a possibility of contracting at least one of the target cancers, or an animal that was affected with at least one of the target cancers in the past.

The quantification results for the target miRNA obtained in the quantification step (S11) can be used in order to detect at least one of the target cancers in a subject.

For example, the analytical method according to the second embodiment can further include a determination step (S12) that can be carried out after the quantification step (S11) as shown in part (b) of FIG. 2. In the determination step (S12), when a quantitative value of the target miRNA is high, the subject can be determined to have contracted at least one of the target cancers. The level of the quantitative value is determined based on, for example, the quantitative value or the threshold value of the target miRNA in a control obtained in advance.

The control according to the second embodiment refers to, for example, an individual that is not affected with any of the target cancers. The control may be a normal subject or an individual that has contracted another disease; however, the control is preferably an individual that has contracted another cancer. The other cancer is a cancer that is classified to be other than the target cancers. The other cancer is, for example, uterine sarcoma.

The threshold value is, for example, a quantitative value of the target miRNA, by which the quantitative values for the subject that has contracted cancer and the quantitative values for the control that is not affected with cancer can be divided; however, the threshold value is preferably a quantitative value by which the quantitative values for the subject that has contracted a target cancer and the quantitative values for the control that has contracted uterine sarcoma can be divided. The threshold value can be determined, for example, from the quantitative values of the target miRNA obtained from the same or similar sample originating from a standard subject, for which it is known whether the standard subject has contracted cancer or the type of the contracted cancer is known, or a sample containing an established cell line of a cancer. The threshold value is not limited and can be determined according to the quantification method used, the type of the sample, measurement conditions, and the like.

The quantitative value or the threshold value for the control may be determined from findings in the past, such as literature.

When the quantitative value obtained in the quantification step (S11) is high, it can be determined that the subject is not affected with uterine sarcoma but has contracted at least one of the target cancers. In other words, according to the present analytical method, it is possible to distinguish between a subject that has contracted a target cancer and a subject that has contracted uterine sarcoma or a subject that is not affected with cancer.

By carrying out the determination step (S12) based on the quantitative value of the control for uterine sarcoma and/or the threshold value for dividing between a target cancer and uterine sarcoma, the presence or absence of contraction of the target cancer can be determined by more accurately distinguishing between the target cancer and uterine sarcoma cancer.

According to the analytical method of the second embodiment described above, it is possible to conveniently determine the presence or absence of contraction of at least one of the target cancers in a subject by quantifying one kind of target miRNA (hsa-miR-1301-3p).

As shown in part (c) of FIG. 2, the analytical method of the second embodiment may include, after the quantification step (S11), a determination step (S13) of determining the presence or absence of the prognosis or recurrence of a target cancer in a subject from the quantification results. Furthermore, after the determination step (S12) and/or the determination step (S13), the analytical method may include a selection step (S14) of selecting the type of a therapeutic method or the type of a medicine to be applied to the subject according to the results of the determination step.

According to still another embodiment, there is also provided an analytical method for assisting in determining the presence or absence of contraction of at least one of the target cancers in a subject, the analytical method including quantifying a target miRNA in a sample originating from the subject (quantification step (S11)). According to the present method, more accurate information for implementing determining the presence or absence of contraction of a target cancer in a subject, determining the prognosis, determining the presence or absence of recurrence, selecting a therapeutic method or a medicine to be applied to the subject, or the like can be acquired.

According to the second embodiment, there is also provided a marker for detecting a target cancer, the marker including hsa-miR-1301-3p.

According to the second embodiment, a kit for detecting a target cancer is provided. The kit according to the second embodiment includes a nucleic acid similar to that described in the first embodiment; however, the primer set may be that intended for a LAMP method. The kit may further include a reagent required for the quantification step. The kit according to the second embodiment may be provided as a diagnostic composition or a diagnostic agent for detecting at least one of the target cancers. Furthermore, according to the second embodiment, there is also provided the use of at least one of the above-described nucleic acids for the production of this diagnostic composition or a diagnostic agent.

The quantitative value of hsa-miR-1301-3p obtained by the LAMP method can be used for universally detecting a target cancer. For example, when the quantitative value of hsa-miR-1301-3p for cells obtained from a subject is equal to or higher than the threshold value, the subject can be determined to have contracted at least one of the target cancers. Furthermore, it is also possible to determine more accurately whether the subject has contracted any one of the target cancers, by quantifying another marker that is specific to each of the target cancers.

The other marker is, for example, a marker other than hsa-miR-1301-3p, intended for detecting at least one of the target cancers.

For example, hsa-miR-92a-3p, the expression level of which is high in prostate cancer, can be used in combination. hsa-miR-92a-3p is an miRNA having the following sequence: UAUUGCACUUGUCCCGGCCUGU (SEQ ID NO: 2). When the quantitative values of both the target miRNA and hsa-miR-92a-3p are high, it can be determined that the subject has contracted prostate cancer.

Furthermore, hsa-miR-122a-5p, the expression level of which is high in pancreatic cancer, can be used in combination. hsa-miR-122a-5p is an miRNA having the following sequence: UGGAGUGUGACAAUGGUGUUUG (SEQ ID NO: 3). When the quantitative values of both the target miRNA and hsa-miR-122a-5p are high, it can be determined that the subject has contracted pancreatic cancer.

It is preferable that the quantitative values of the other markers are acquired by methods similar to that of the quantification step (S1).

The other marker is not limited to those described above, and any marker known as a marker for detecting any of the target cancers can all be used. The other marker does not have to be a miRNA and may be a DNA, a peptide, a protein, or the like.

The kit in this example may further include an RT primer, an EL primer, an amplification primer set, and/or a nucleic acid probe, which are intended for detecting the above-described other marker.

### • Third embodiment

In a third embodiment, a preferred example of a step of quantifying a target miRNA will be described. In this example, the target miRNA is specifically subjected to reverse transcription, an artificial sequence is added to elongate the sequence, the elongation product is amplified by a LAMP method, and the amplification product thus obtained is detected. In the following description, the sequence of the target miRNA (SEQ ID NO: 1) is also referred to as "first sequence".

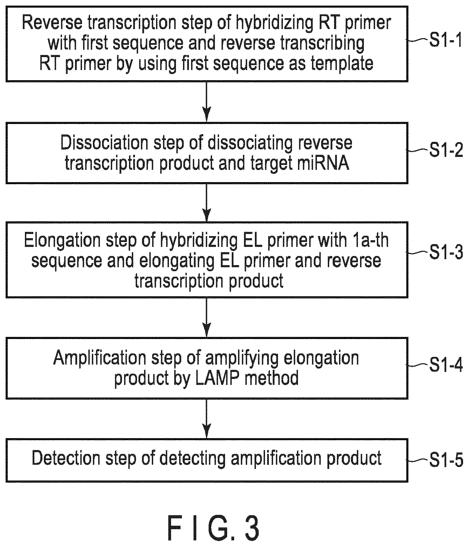

A quantification step (S1) includes, for example, the following steps shown in FIG. 3:
(S1-1) a reverse transcription step of hybridizing a first primer part of a reverse transcription (RT) primer containing the first primer part, which is hybridized with the first sequence of the target miRNA, and a first LAMP recognition sequence, with the first sequence, subjecting the first sequence to reverse transcription, and obtaining a reverse transcription product containing the cDNA (1a-th sequence) of the target miRNA;
(S1-2) a dissociation step of dissociating the reverse transcription product from the target miRNA;
(S1-3) an elongation step of hybridizing a second primer part of an elongation (EL) primer containing the second primer part, which is hybridized with the 1a-th sequence, and a second LAMP recognition sequence, with the 1a-th sequence of the reverse transcription product, elongating the EL primer and the reverse transcription product by using each other as templates, and obtaining an elongation product containing a complementary sequence of the 1a-th sequence 5 (that is, DNA sequence corresponding to the first sequence);
(S1-4) an amplification step of amplifying the elongation product by a LAMP reaction and obtaining an amplification product; and
(S1-5) a detection step of detecting the obtained amplification product.

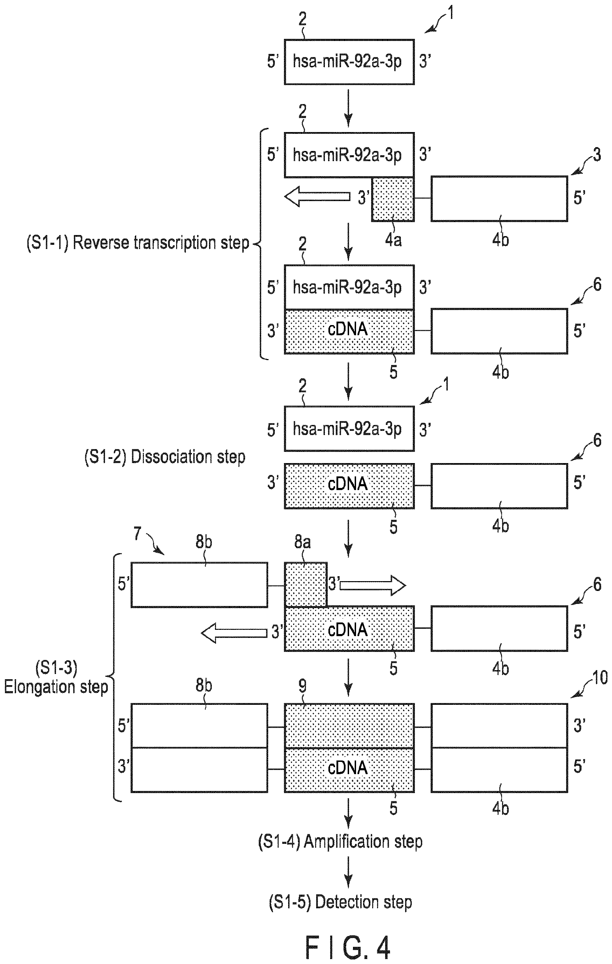

In the following description, each step will be described in detail using FIG. 4.

In the reverse transcription step (S1-1), for example, a reverse transcription solution including an RT primer 3, a reverse transcriptase, a salt, a substrate such as deoxynucleoside triphosphate (dNTP) (if necessary, a thickening agent, a buffer material for pH adjustment, a surfactant, an ion that increases annealing specificity, and/or an ion serving as a cofactor of the reverse transcriptase, as reactive reagents), and the like is used. As the reverse transcriptase, for example, M-MuLV reverse transcriptase, AMV reverse transcriptase, transcriptor reverse transcriptase, SuperScript (registered trademark) transcriptor reverse transcriptase, or MultiScribe reverse transcriptase can be used.

An RT primer 3 includes a first primer part 4a and a first LAMP recognition sequence 4b. The first primer part 4a is a nucleic acid sequence that is hybridized with a first sequence 2 and thereby works as a primer for reverse transcribing the first sequence 2 to produce cDNA (1a-th sequence 5). The first primer part 4a has, for example, a complementary sequence of at least five consecutive base sequences including the 3'-end of the first sequence 2.

The first LAMP recognition sequence 4b is a nucleic acid base sequence containing a sequence to which an amplification primer binds (recognition sequence) in the amplification step (S1-4). The recognition sequence may be a recognition sequence generally used with respect to a primer for LAMP amplification. For example, the first LAMP recognition sequence 4b may contain a B1 sequence, a B2 sequence, a B3 sequence, and/or an LB sequence in order from the side of the first primer part 4a toward the 5'-end.

The concentration of the RT primer 3 in the reverse transcription solution is preferably 5 to 50 nM. When the concentration is in this concentration range, specificity and efficiency of the reaction can be further enhanced.

In the reverse transcription step (S1-1), it is preferable that the reverse transcription solution and a sample are mixed and maintained at, for example, about 10°C to 55°C. Accordingly, the first primer part 4a is hybridized with the first sequence 2, the first sequence 2 is reverse transcribed, and a reverse transcription product 6 containing the cDNA of the target miRNA 1 (1a-th sequence 5) is obtained.

The dissociation step (S1-2) can be carried out by, for example, heating the reaction liquid after reverse transcription to 80°C to 100°C. By maintaining the reaction liquid under these conditions, the reverse transcription product 6 and the target miRNA 1 are dissociated.

In the elongation step (S1-3), for example, an elongation solution including an EL primer 7, a DNA polymerase, a salt, a substrate such as dNTP (if necessary, a thickening agent, a buffer material for pH adjustment, a surfactant, and ions), and the like as well as the reverse transcription product 6, is used.

The EL primer 7 contains a second primer part 8a and a second LAMP recognition sequence 8b. The second primer part 8a is a nucleic acid sequence that is hybridized with the 1a-th sequence 5 and works as a primer for producing a complementary sequence 9 thereof. The second primer part 8a has, for example, a complementary sequence of at least five consecutive base sequences containing the 3'-end of the 1a-th sequence 5.

The second LAMP recognition sequence 8b is a nucleic acid base sequence containing a sequence to which an amplification primer binds (recognition sequence) in the amplification step (S1-4). For example, the second LAMP recognition sequence 8b may contain sequence F1, sequence F2, sequence F3, and/or sequence LF in order from the side of the second primer part 8a toward the 5'-end.

The concentration of the EL primer 7 in the elongation solution is preferably 5 to 100 nM. When the concentration is in this concentration range, specificity and efficiency of the reaction can be further enhanced.

With regard to the elongation step (S1-3), it is preferable that the elongation solution and a reaction liquid including the reverse transcription product 6 after the dissociation step are mixed and maintained at about 10°C to 80°C. Accordingly, the second primer part 8a of the EL primer 7 is hybridized with the 1a-th sequence 5 of the reverse transcription product 6, the EL primer 7 and the reverse transcription product 6 are elongated by using each other as templates, and an elongation product 10 containing a complementary sequence 9 of the 1a-th sequence 5 (that is, DNA sequence corresponding to the first sequence) is obtained.

The first primer part 4a and the second primer part 8a may be configured to contain DNA only or may contain LNA and/or PNA. As more of these are included, the binding force of hybridization can be made stronger. Therefore, the number of LNA and/or PNA may be determined according to the desired Tm value with the sequence to be hybridized. For example, when LNA and/or PNA is included, the reverse transcription step (S1-1) and the elongation step (S1-3) can be carried out at higher temperatures, and non-specific binding can be suppressed. As a result, it is possible to perform reverse transcription and elongation of the target miRNA with higher accuracy.

It is preferable that the first LAMP recognition sequence 4b and the second LAMP recognition sequence 8b contain recognition sequences in the following combinations of (1) to (7). Here, the order of each of the following recognition sequences is the order from the side of the first primer part 4a or the second primer part 8a toward to the 5'-end. The term "c" means a complementary sequence of the sequence described immediately before the letter. For example, the term "LBc sequence" means a complementary sequence of an LB sequence. A term "dummy sequence" is a nucleic acid sequence having a base sequence different from the 1a-th sequence, an F2 sequence, an F1 sequence, an LF sequence, a B1 sequence, a B2 sequence, an LB sequence, and complementary sequences thereof.
(1) The first LAMP recognition sequence 4b contains the B2 sequence, and
   the second LAMP recognition sequence 8b contains a B1c sequence, the F1 sequence, and the F2 sequence.
   (In this case, at least a part of a complementary sequence 9 of the 1a-th sequence is referred to the LB sequence.)
(2) The first LAMP recognition sequence 4b contains the LBc sequence and the B2 sequence, and
   the second LAMP recognition sequence 8b contains the B1c sequence, the F1 sequence, and the F2 sequence.
(3) The first LAMP recognition sequence 4b contains a dummy sequence and the B2 sequence, and
   the second LAMP recognition sequence 8b contains the B1c sequence, the F1 sequence, and the F2 sequence.
(4) The first LAMP recognition sequence 4b contains the B1 sequence and the B2 sequence, and
   the second LAMP recognition sequence 8b contains the F1 sequence, the LFc sequence, and the F2 sequence.
(5) The first LAMP recognition sequence 4b contains the B2 sequence, and
   the second LAMP recognition sequence 8b contains the F1 sequence, the LFc sequence, and the F2 sequence.
(6) The first LAMP recognition sequence 4b contains the LBc sequence and the B2 sequence, and
   the second LAMP recognition sequence 8b contains the F1 sequence and the F2 sequence.
(7) The first LAMP recognition sequence 4b contains the B1 sequence and the B2 sequence, and
   the second LAMP recognition sequence 8b contains the LFc sequence and the F2 sequence.

A spacer sequence may be present between the first primer part 4a and the first LAMP recognition sequence 4b of the RT primer 3, between the second primer part 8a and the second LAMP recognition sequence 8b of the EL primer 7, and/or between the respective recognition sequences included in the respective LAMP recognition sequences. The spacer sequence is a nucleic acid sequence that is different from the sequences of the 1a-th sequence, the respective recognition sequences, and complementary sequences thereof and does not adversely affect an amplification reaction of the elongation product that will be described below. The spacer sequence may be configured to contain DNA only or may contain LNA and/or PNA. For example, the spacer sequence contains 1 base to 16 bases and is preferably a poly T sequence, a poly A sequence, or the like.

The amplification step (S1-4) is carried out by using a LAMP method. In this step, for example, an amplification solution including an amplification primer set, a chain-substitution type DNA polymerase, a salt, a substrate such as dNTP (if necessary, a thickening agent, a buffer material for pH adjustment, a surfactant, and ions), and the like is used.

The type and sequence of the amplification primer set are selected according to the sequences of the first LAMP recognition sequence 4b and the second LAMP recognition sequence 8b. For example, the amplification primer set includes an FIP primer and a BIP primer corresponding to the first LAMP recognition sequence 4b and the second LAMP recognition sequence 8b. If necessary, an F3 primer, a B3 primer, and/or a loop primer such as an LF primer or an LB primer may be further included.

When the combination of the LAMP recognition sequences of the RT primer 3 and the EL primer 7 is set to the above-described combinations (1) to (7), it is preferable that the amplification primer set is configured to employ the following combinations. Incidentally, the following (1) to (7) correspond to the above-described (1) to (7), respectively.
(1) An FIP primer containing the F2 sequence and the F1c sequence,
   a BIP primer containing the B2 sequence and the B1c sequence, and
   an LB primer containing the complementary sequence 9 of the 1a-th sequence.
(2) An FIP primer containing the F2 sequence and the F1c sequence,
   a BIP primer containing the B2 sequence and the B1c sequence, and
   an LB primer containing the LB sequence.
(3) An FIP primer containing the F2 sequence and the F1c sequence,
   a BIP primer containing the B2 sequence and the B1c sequence, and
   an LB primer containing the complementary sequence 9 of the 1a-th sequence.
(4) An FIP primer containing the B2 sequence and the B1c sequence,
   a BIP primer containing the F2 sequence and the F1c sequence, and
   an LF primer containing the LF sequence.
(5) An FIP primer containing the F2 sequence and the F1c sequence,
   a BIP primer containing the B2 sequence and the complementary sequence 9 of the 1a-th sequence, and
   an LF primer containing the LF sequence.
(6) An FIP primer containing the F2 sequence and the F1c sequence,
   a BIP primer containing the B2 sequence and the complementary sequence 9 of the 1a-th sequence, and
   an LB primer containing the LB sequence.
(7) An FIP primer containing the F2 sequence and the 1a-th sequence 5,
   a BIP primer containing the B2 sequence and the B1c sequence, and
   an LF primer containing the LF sequence.

It is more preferable to employ the above-described combination of (1). Furthermore, in the case of adopting the combination of (1), it is preferable to use, for example, primer sets A to D or primer set J shown in the following Table 1 as the RT primer 3, the EL primer 7, and the amplification primer set.

### Table 1-1

**Table 1**

| Primer set A | | |
|---|---|---|
| Primer name | SEQ ID NO | Sequence (5' -3' ) |
| RT | 4 | AGGATCGTACGAGACCGAAGGAAGTCACT |
| EL | 5 | GAGTCGTCGCCAGTACCTCGGTTGGAGCTGAATTTTGCGTGGA CCGACGGCTTGATGCAGGGTTGCAGCTGCCTGGG |
| FIP | 6 | ACGCAAAATTCAGCTCCAACCGTTGAGTCGTCGCCAGTACCT |
| BIP | 7 | GGACCGACGGCTTGATGCAAGGATCGTACGAGACCGAAG |
| LB | 8 | TGCCTGGGAGTGACTTC |

| Primer set B | | |
|---|---|---|
| Primer name | SEQ ID NO | Sequence (5' -3' ) |
| RT | 9 | CTGCAACGTTGAACATGCGGAAGTCACT |
| EL | 10 | CTTCGTGCAGATGGCTATGCGGCTAGAATACGGCACGACTGCTG ATGTGTCTGATAGCGGCACGGGGTTGCAGCTGCCTGGG |
| FIP | 11 | GCAGTCGTGCCGTATTCTAGCCCTTCGTGCAGATGGCTATGC |
| BIP | 12 | TGATGTGTCTGATAGCGGCACGCTGCAACGTTGAACATGCG |
| LB | 8 | TGCCTGGGAGTGACTTC |

| Primer set C | | |
|---|---|---|
| Primer name | SEQ ID NO | Sequence (5' -3' ) |
| RT | 13 | TCTGCTCTGGTTGCTCTCATGAAGTCACT |
| EL | 14 | ATCTGCTGCACCACTGAAAGCTCGGTAGACGCATAGTCGCGAGT CGGGAACCCTGTGGGTTTGGGTTGCAGCTGCCTGGG |
| FIP | 15 | CGCGACTATGCGTCTACCGAGATCTGCTGCACCACTGAAAG |
| BIP | 16 | AGTCGGGAACCCTGTGGGTTTTCTGCTCTGGTTGCTCTCAT |
| LB | 8 | TGCCTGGGAGTGACTTC |

### Table 1-2

**Table 1**

| Primer set D | | |
|---|---|---|
| Primer name | SEQ ID NO | Sequence (5' -3' ) |
| RT | 17 | GGCGCCGAAACAATATTCCTGAAGTCACT |
| EL | 18 | GATCTAGAAGGCCGCCAGTCGTTCAGCCTACGGCCGTTGTCATC CGTAGCAGGACGCTCAGGGTTGCAGCTGCCTGGG |
| FIP | 19 | AACGGCCGTAGGCTGAACGGATCTAGAAGGCCGCCAGT |
| BIP | 20 | GTCATCCGTAGCAGGACGCTCAGGCGCCGAAACAATATTCCT |
| LB | 8 | TGCCTGGGAGTGACTTC |

| Primer set J | | |
|---|---|---|
| Primer name | SEQ ID NO | Sequence (5' -3' ) |
| RT | 45 | ATGAGAGGACATACCTCCGCGAAGTCACT |
| EL | 46 | TCAGTCGGGGACAATCCGGTCACTAGAGTCCCGGTCGGTTCGG CGAATAGGGCTCCCAGTAGGGTTGCAGCTGCCTGGG |
| FIP | 47 | AACCGACCGGGACTCTAGTGACTCAGTCGGGGACAATCCG |
| BIP | 48 | CGGCGAATAGGGCTCCCAGTAATGAGAGGACATACCTCCGC |
| LB | 8 | TGCCTGGGAGTGACTTC |

Particularly, it is preferable to use the primer set A or J. These primer sets have particularly excellent stability compared to other primer sets. Primer set J has superior stability and is more preferable. "Having excellent stability" implies that when detection is performed several times, variance is not likely to occur in the results, non-specific amplification is not likely to occur, and more reliable results are obtained.

The sequence of each of the primers is not limited to that shown in the above-described table, and the above-described sequence having deletion, substitution, addition, or insertion of one or two bases therein can also be used.

The concentration of the FIP primer, the BIP primer, or the LB primer in the amplification solution is preferably 0.8 µM to 2.4 µM for the FIP primer and the BIP primer, and 0.4 µM to 1.2 µM for the LB primer. When the concentration is in this concentration range, specificity and efficiency of the reaction can be further enhanced.

In the amplification step (S1-4), a mixture of a solution containing the elongation product 10 and the amplification solution is maintained under isothermal amplification reaction conditions. For example, the isothermal amplification conditions may be set to maintain isothermal conditions at a temperature of 50°C to 75°C for 30 to 90 minutes or the like; however, it is preferable that the temperature is 60°C to 70°C. As a result, the elongation product 10 is amplified, and an amplification product containing the cDNA of the target miRNA 1 (1a-th sequence 5) and a complementary sequence thereof is obtained.

In a detection step (S1-5), it is preferable that a signal that changes with an increase in the amplification product in the amplification step (S1-4) is detected over time, the rise time of the signal is measured, and as a result, quantification of the target miRNA is performed. The term "over time" may be continuous, or mean that detection may be performed at a plurality of time points at a desired time interval.

As the signal, it is preferable to use, for example, the turbidity of a reaction liquid, or an optical signal or an electrochemical signal from the reaction liquid. In the case of using an optical signal, for example, it is preferable to perform an amplification reaction in the presence of a labeling substance that produces an optical signal that changes with an increase in the amplification product. In this case, the labeling substance is, for example, an indicator such as a fluorescent reagent or an intercalator. In the case of using an electrochemical signal, for example, it is preferable to perform an amplification reaction in the presence of a labeling substance that produces an electrochemical signal that changes with an increase in the amplification product. In this case, the labeling substance is, for example, a redox probe.

Detection is preferably performed by, for example, using a detection device. The detection device includes, for example, a chip. The chip is an amplification detection unit that amplifies the target miRNA and detects the amplification product, and includes, for example, a substrate and one or more detection regions provided on one surface of the substrate. For example, the reverse transcription step (S1-1) to the amplification step (S1-4) can be carried out on the one surface, and when an amplification product is present, a signal can be detected at the detection region. Furthermore, the detection device can further include a calculation unit that calculates a quantitative value of the target miRNA from the results of detection obtained at the amplification detection unit.

In the case of using an optical signal, the detection region is, for example, an optical sensor. In the case of using an electrochemical signal, the detection region is, for example, an electrode. The electrode is preferably, for example, gold due to its favorable sensitivity.

In the vicinity of the detection region, a reverse transcription primer for reverse transcribing the target miRNA, an elongation primer for elongating the target miRNA, and/or an amplification primer set for amplifying the target miRNA may be releasably immobilized.

Furthermore, in the detection step (S1-5), a nucleic acid probe may be used. The nucleic acid probe has a sequence that is hybridized with the amplification product, and the amplification product can be detected by detecting the hybridization of the nucleic acid probe and the amplification product. For example, the nucleic acid probe may be immobilized in the vicinity of the detection region of the detection device.

Next, the target miRNA in the sample is quantified from the rise time of the signal obtained in the detection step (S1-5). For example, it can be determined that as the rise time is earlier, the abundance of the target miRNA is larger. For example, it is preferable to perform quantification of the target miRNA in the sample by using a calibration curve showing the relationship between the rise time and the abundance of the target miRNA.

According to the quantification step of the third embodiment described above, the target miRNA can be subjected to reverse transcription, elongation, amplification, and detection more specifically and efficiently. Therefore, by performing the quantification step of the third embodiment in place of the quantification step (S1) of the first embodiment and the quantification step (S11) of the second embodiment, the presence or absence of contraction of cancer, particularly at least one of the target cancers, can be determined more accurately.

According to still another embodiment, the extraction efficiency of RNA may vary depending on the type of cells. Therefore, it is preferable to correct the quantitative value obtained in the quantification step based on the RNA extraction efficiency before using the quantitative value in the determination step. The correction can be carried out, for example, as follows. A known amount of standard miRNA is added in advance to a sample. For example, standard miRNA is added (spike-in) to a reagent for extracting RNA from the sample. Subsequently, the standard miRNA is extracted together with the target miRNA, and quantification of the standard miRNA is performed together with the quantification of the target miRNA. Subsequently, the extraction efficiency is evaluated from the quantitative value of the standard miRNA, and the quantitative value of the target miRNA can be corrected.

It is preferable that quantification of the standard miRNA is carried out in the same manner as in the quantification of the target miRNA, for example, by the method shown in FIG. 4 using a dedicated primer set.

Regarding the standard miRNA, it is preferable to use a miRNA that does not exist in the subject. As the standard miRNA, for example, cel-miR-39-3p, which is present in nematodes, can be used. cel-miR-39-3p is an miRNA having the following sequence:
UCACCGGGUGUAAAUCAGCUUG (SEQ ID NO: 49).

cel-miR-39-3p can be quantified by using a primer set K described in the following Table 2.

### Table 2

**Table 2. Primer set K for ce|-miR-39-3p**

| Primer name | SEQ ID NO | Sequence (5' -3' ) |
|---|---|---|
| RT | 50 | TCAGGTTCAGATCATCCCCAGGGTCTGCAGACGTTTCTCCAAGCTGAT |
| EL | 51 | ATACGGTCATTGCTGCTCCTACGAGGCAGAACAATGCCCTCGGGTAGT TGAGGCACCATGACGTGGGTCACCGGGTGTAA |
| FIP | 52 | GAGGGCATTGTTCTGCCTCGTATTTTAATACGGTCATTGCTGCTCC |
| BIP | 53 | GGTAGTTGAGGCACCATGACGTTCAGGTTCAGATCATCCCCA |
| LB | 54 | GGTGTAAATCAGCTTG |

The sequence of each of the primers is not limited to that shown in the above-described table, and the above-described sequence having deletion, substitution, addition, or insertion of one or two bases therein can also be used.

For example, a corrected value is obtained by dividing the quantitative value of the target miRNA by the quantitative value of the standard miRNA. By using the corrected value in the subsequent determination step, more accurate determination can be carried out.

Also with regard to the method of the third embodiment, a kit for determining the presence or absence of contraction of cancer or a target cancer is provided. It is preferable that this kit includes, for example, any of the above-described RT primer 3, EL primer 7, amplification primer set, and/or nucleic acid probe. For example, it is preferable that the kit includes at least one selected from the primer sets A to D and J shown in the above-described Table 1.

Furthermore, also with regard to the method of the third embodiment, the other marker explained in reference to the second embodiment can be used in combination. In that case, it is preferable that the other marker is also quantified by the method of the third embodiment. Furthermore, in that case, the kit may further include an RT primer, an EL primer, and an amplification primer set for the other marker.

For example, in the case of using hsa-miR-92a-3p in combination, it is preferable that the kit further includes any of primer sets E to G described in the following Table 3. With regard to the LB primer, either SEQ ID NO: 25 or SEQ ID NO: 26 can be used for any of the primer sets.

### Table 3

**Table 3**

| Primer set E | | |
|---|---|---|
| Primer name | SEQ ID NO | Sequence (5' -3' ) |
| RT | 21 | GGAGGCGACACGAGTTCTACAGGCCG |
| EL | 22 | GGCTGTGCAGAGATAGGTGGTACGAAGCAGCCTTCTCGG CTCGGCTATCTACGCGTTAAGCGGGGTATTGCACTTGTCC |
| FIP | 23 | GCCGAGAAGGCTGCTTCGTAGGCTGTGCAGAGATAGGTG |
| BIP | 24 | TCGGCTATCTACGCGTTAAGCGGGAGGCGACACGAGTTCT |
| LB | 25 | ACTTGTCCCGGCCTGT |
| LB | 26 | TTGTCCCGGCCTGTAGA |

| Primer set F | | |
|---|---|---|
| Primer name | SEQ ID NO | Sequence (5' -3' ) |
| RT | 27 | GGCGCCGAAACAATATTCCTACAGGCCG |
| EL | 28 | GATCTAGAAGGCCGCCAGTCGTTCAGCCTACGGCCGTTGT CATCCGTAGCAGGACGCTCAGGGTATTGCACTTGTCC |
| FIP | 29 | AACGGCCGTAGGCTGAACGGATCTAGAAGGCCGCCAGT |
| BIP | 30 | GTCATCCGTAGCAGGACGCTCAGGCGCCGAAACAATATTCCT |
| LB | 25 | ACTTGTCCCGGCCTGT |

| Primer set G | | |
|---|---|---|
| Primer name | SEQ ID NO | Sequence (5' -3' ) |
| RT | 31 | CTGCAACGTTGAACATGCGACAGGCCG |
| EL | 32 | TTCGTGCAGATGGCTATGCGGCTAGAATACGGCACGACTG CTGATGTGTCTGATAGCGGCACGGGGTATTGCACTTGTCC |
| FIP | 33 | GCAGTCGTGCCGTATTCTAGCCCTTCGTGCAGATGGCTATGC |
| BIP | 34 | TGATGTGTCTGATAGCGGCACGCTGCAACGTTGAACATGCG |
| LB | 25 | ACTTGTCCCGGCCTGT |

The sequence of each of the primers is not limited to that shown in the above-described table, and the above-described sequence having deletion, substitution, addition, or insertion of one or two bases therein can also be used.

In the case of using hsa-miR-122-5p in combination, it is preferable to use primer set H or I described in the following Table 4. With regard to the LB primer, either SEQ ID NO: 39 or SEQ ID NO: 40 can be used for any of the primer sets.

### Table 4

**Table 4**

| Primer set H | | |
|---|---|---|
| Primer name | SEQ ID NO | Sequence (5' -3' ) |
| RT | 35 | CGGAACGGCATAAAAAGCCCAAACACCA |
| EL | 36 | GAATGTGACCACGCGGATACAGACTTTCGATCCACGCTGG GACCGAGGCCAGACTCTACCTGGGTGGAGTGTGACAA |
| FIP | 37 | CCAGCGTGGATCGAAAGTCTGTGAATGTGACCACGCGGAT |
| BIP | 38 | GACCGAGGCCAGACTCTACCTCGGAACGGCATAAAAAGCC |
| LB | 39 | GTGTGACAATGGTGTTTG |
| LB | 40 | TGACAATGGTGTTTGGGC |

| Primer set I | | |
|---|---|---|
| Primer name | SEQ ID NO | Sequence (5' -3' ) |
| RT | 41 | GGCGCCGAAACAATATTCCTCAAACACCA |
| EL | 42 | GATCTAGAAGGCCGCCAGTCGTTCAGCCTACGGCCGTTGT CATCCGTAGCAGGACGCTCAGGGTGGAGTGTGACAA |
| FIP | 43 | AACGGCCGTAGGCTGAACGGATCTAGAAGGCCGCCAGT |
| BIP | 44 | GTCATCCGTAGCAGGACGCTCAGGCGCCGAAACAATATTCCT |
| LB | 39 | GTGTGACAATGGTGTTTG |

The sequence of each of the primers is not limited to that shown in the above-described table, and the above-described sequence having deletion, substitution, addition, or insertion of one or two bases therein can also be used.

When the primer sets represented in Tables 3 and 4 are used, hsa-miR-92a-3p and hsa-miR-122-5p can be quantified more specifically. As a result, the detection accuracy for prostate cancer and pancreatic cancer is further enhanced. The primer set for the other marker included in the kit is not limited to the above-described primer sets, and any other primer sets can also be included in the kit.

Alternatively, since the quantification step and kit of the third embodiment enable detection and quantification of the target miRNA more specifically, even in a case where the quantification step and kit are used for the detection of another disease where the target miRNA is used as a marker, detection thereof can be carried out more accurately. Examples of the other disease include neuropathy, cognitive impairment, hepatitis, heart diseases, and autoimmune diseases such as rheumatism.

Furthermore, the kit according to the third embodiment may include a primer set or a nucleic acid probe for quantifying an miRNA for correction. For example, the kit may further include primer set K shown in Table 2.

### [Examples]

Hereinafter, experiments carried out using the analytical methods or kits of the embodiments will be described.

Example 1. Quantification of hsa-miR-1301-3p in healthy subjects and cancer patients using qRT-PCR method

Blood sera of 114 specimens in total obtained from 29 specimens of healthy subjects, 15 specimens of breast cancer patients, 6 specimens of colorectal cancer patients, 5 specimens of stomach cancer patients, 3 specimens of lung cancer patients, 5 specimens of ovarian cancer patients, 8 specimens of pancreatic cancer patients, 5 specimens of bile duct cancer patients, 5 specimens of esophageal cancer patients, 5 specimens of liver cancer patients, 5 specimens of brain tumor patients, 4 specimens of urinary bladder cancer patients, 5 specimens of prostate cancer patients, 5 specimens of sarcoma patients, 4 specimens of uterine body cancer patients, and 5 specimens of uterine sarcoma patients were prepared.

### (Quantification of hsa-miR-1301-3p)

RNA was extracted from all the blood sera. Extraction was performed by using NucleoSpin (registered trademark) miRNA Plasma (trade name, manufactured by Takara Bio, Inc.). Synthetic RNA of cel-miR-39-3p was spiked in, and extraction was performed.

Next, short stranded RNA present in an extracted sample was reverse transcribed, and cDNA was synthesized and amplified. Synthesis was performed using TaqMan (registered trademark) Advanced miRNA cDNA Synthesis Kit (trade name, manufactured by Thermo Fisher Scientific, Inc.) according to the manual of TaqMan (registered trademark) Advanced miRNA Assay (trade name, manufactured by Thermo Fisher Scientific, Inc.).

Subsequently, TaqMan PCR was performed by using TaqMan (registered trademark) Fast Advanced Master Mix (trade name, manufactured by Thermo Fisher Scientific, Inc.) and TaqMan (registered trademark) Advanced miRNA Assay ID: 477897_mir, and a Ct value was measured. Together with the sample, a synthetic RNA set forth in SEQ ID NO: 1 at different concentrations (10³, 10⁴, 10⁵, or 10⁶ copies/pL) was used to perform a reaction in the same manner, and a calibration curve was obtained.

### (Quantification of cel-miR-39-3p)

cel-miR-39-3p (SEQ ID NO: 49) in the above-described blood serum samples was quantified. Chain lengthening and amplification of cel-miR-39-3p were performed by methods similar to those used for hsa-miR-1301-3p. cel-miR-39-3p was caused to react at concentrations of 10³, 10⁴, 10⁵, or 10⁶ copies/pL to produce a calibration curve. A quantitative value of cel-miR-39-3p was obtained by making a comparison with the calibration curve.

### (Calculation of corrected value)

Next, the quantitative value of hsa-miR-1301-3p was divided by the quantitative value of cel-miR-39-3p, and a corrected value was obtained.

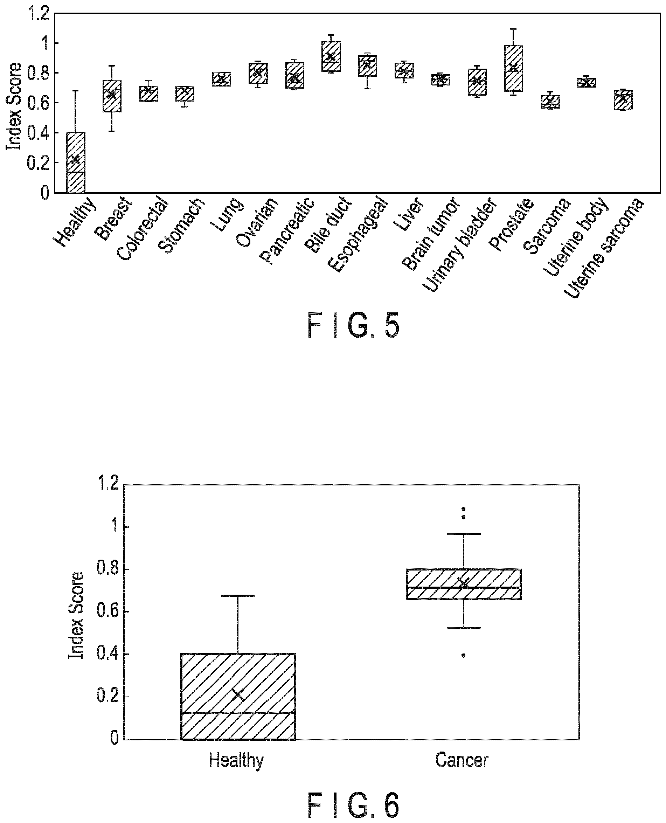

FIG. 5 shows a box-and-whisker plot of corrected values of the quantitative values of hsa-miR-1301-3p in healthy subjects and various cancer patients. The corrected values were distributed in the range of 0 to about 0.4 in healthy subjects and were distributed in the range of about 0.55 to about 1 in cancer patients. From these results, it was found that the abundance of hsa-miR-1301-3p in the blood serum was large in cancer patients.

FIG. 6 is a box-and-whisker plot showing the results of classifying the compensated values into a healthy subject group and a cancer patient group. The cancer patient group was a group of combining the results of all the cancer patients other than healthy subjects, and the results were distributed at higher values than that of healthy subjects. The AUC (Area Under Curve) dividing between the healthy subject group and the cancer patient group was 0.98.

Therefore, it was found that hsa-miR-1301-3p had high performance as a marker for distinguishing and detecting between a healthy subject and a cancer patient.

Example 2. Quantification of hsa-miR-1301-3p in healthy subjects and cancer patients using LAMP method

### (Quantification of hsa-miR-1301-3p)

Blood sera obtained from specimens similar to those used in Example 1 were prepared. Extraction of RNA from the specimens was performed by the same method as in Example 1. 2 µL each of the extracted samples was subjected to reverse transcription under the conditions of a reaction volume of 20 µL, 10 minutes at 16°C, 5 minutes at 42°C, and 5 minutes at 85°C. The composition of the reverse transcription reaction solution was 67 units of MultiScribe (registered trademark) Reverse Transcriptase(*), 1×RT Buffer(*), 0.1 mM of dNTPs(*), 4 U of RNaseOUT (trade name, manufactured by Thermo Fisher Scientific, Inc.), and 10 nM of RT primer. Regarding the components assigned with the symbol "*", all of those included in High-Capacity cDNA Reverse Transcription Kit (trade name, manufactured by Thermo Fisher Scientific, Inc.) were used.

5 µL of an elongation solution was added to the reaction solution after reverse transcription, and then after 2 minutes at 95°C, elongation was performed by applying 20 cycles of 20 seconds at 95°C, 30 seconds at 59°C, and 10 seconds at 72°C. The elongation solution included Deep Vent (exo-) DNA polymerase (0.5 U, New England Biolabs, Ltd.) in 25 µL, and the elongation solution was prepared such that the final concentrations would be 0.2×ThermoPol Buffer (attached to Deep Vent (exo-) DNA polymerase), 0.2 mM of MgSO₄, 0.12 mM of dNTPs, and 10 nM of EL primer.

Next, 1 µL of the elongation product was subjected to LAMP amplification under the conditions of a reaction volume of 25 µL and 60 minutes at 65°C, and the rise time for the fluorescence intensity was measured at the same time. A LAMP amplification solution included 8 U of Tin(exo-) LF DNA polymerase (Optigene) and 0.5 µL of EvaGreen (registered trademark) (Biotium), and the LAMP amplification solution was prepared such that the final concentrations would be 20 mM of Tris-HCl (pH 8.0), 50 mM of KCl, 8 mM of MgSO₄, 10 mM of (NH4)SO4, 0.1% of Tween-20, 0.8 M of betaine, each of dNTPs at 1.4 mM, 1.6 µM of FIP primer, 1.6 µM of BIP primer, and 0.8 µM of LB primer. The fluorescence intensity was measured over time using a real-time PCR apparatus, and the time when the fluorescence intensity exceeds the threshold value was measured.

For the RT primer, EL primer, FIP primer, BIP Primer, and LB primer, the primer set A or primer set J in Table 1 was used.

Together with a sample, the synthetic RNA set forth in SEQ ID NO: 1 at a known concentration was subjected to reverse transcription, elongation, and LAMP amplification by using the above-described primers, and the rise time of the fluorescence intensity was measured similarly. The concentration of the synthetic RNA was set to 0, 10³, 10⁴, 10⁵, and 10⁶ copies/µL for the primer set A, and to 0, 5×10², 5×10³, 5×10⁴, and 5×10⁵ copies/µL for the primer set J. A calibration curve of the number of copies of the RNA set forth in SEQ ID NO: 1 and the rise time was prepared by using these results, and the number of copies of RNA in each specimen was calculated from the rise time by using this calibration curve.

### (Quantification of cel-miR-39-3p)

cel-miR-39-3p (SEQ ID NO: 49) in the above-described blood serum samples was quantified. Extraction, chain lengthening, and amplification of cel-miR-39-3p were performed by methods similar to those used for hsa-miR-1301-3p. Reverse transcription of cel-miR-39-3p was performed by using 20 nM RT primer, and chain lengthening was performed by using 40 nM EL primer. Amplification was performed by using 1.6 µM of FIP primer, 1.6 µM of BIP primer, and 0.8 µM of LB primer.

For the RT primer, EL primer, FIP primer, BIP primer, and LB primer, the primer set K of Table 2 was used.

Also, cel-miR-39-3p was caused to react at concentrations of 10³, 10⁴, 10⁵, or 10⁶ copies/pL to prapare a calibration curve. A quantitative value of cel-miR-39-3p was obtained by making a comparison with the calibration curve.

### (Calculation of corrected value)

Next, the quantitative value of hsa-miR-1301-3p was divided by the quantitative value of cel-miR-39-3p, and a corrected value was obtained.

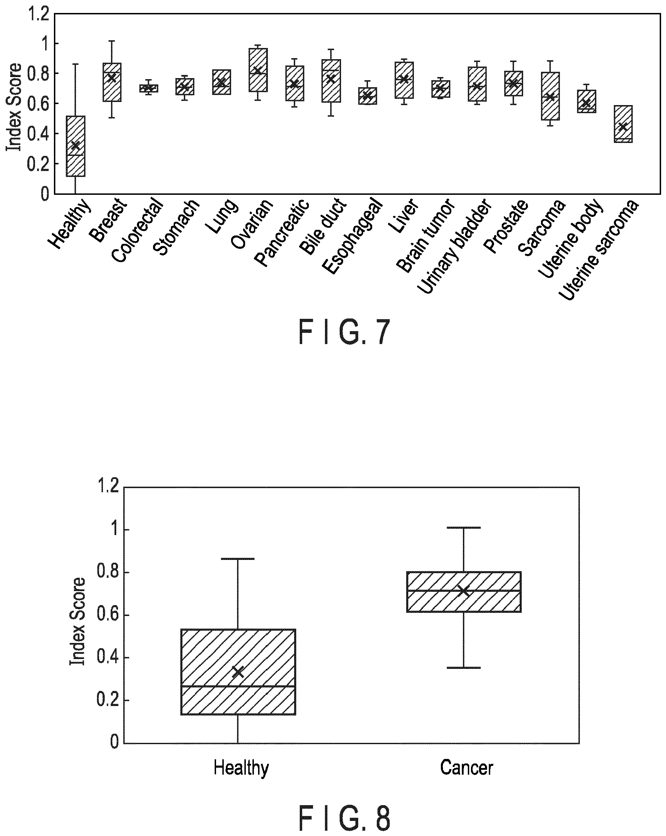

FIG. 7 shows a box-and-whisker plot of corrected values of hsa-miR-1301-3p in healthy subjects and various cancer patients in the case of using the primer set A. Regarding hsa-miR-1301-3p, the corrected values were distributed in the range of about 0.15 to about 0.55 in healthy subjects, while the corrected values were distributed in the range of high values of about 0.35 to about 0.95 in cancer patients. Furthermore, in the patients of the target cancers except for uterine sarcoma, the corrected values were distributed in the range of higher values of about 0.5 to about 0.95. From these results, it was found that the abundance of hsa-miR-1301-3p in the blood serum was large in cancer patients, particularly patients of the target cancers.

FIG. 8 is a box-and-whisker plot showing the results of classifying the corrected values of FIG. 7 into a healthy subject group and a cancer patient group. The cancer patient group was a group of combining the results of all the cancer patients other than healthy subjects, and the results were distributed at higher values than those of healthy subjects. The AUC dividing between the healthy subject group and the cancer patient group was 0.93.

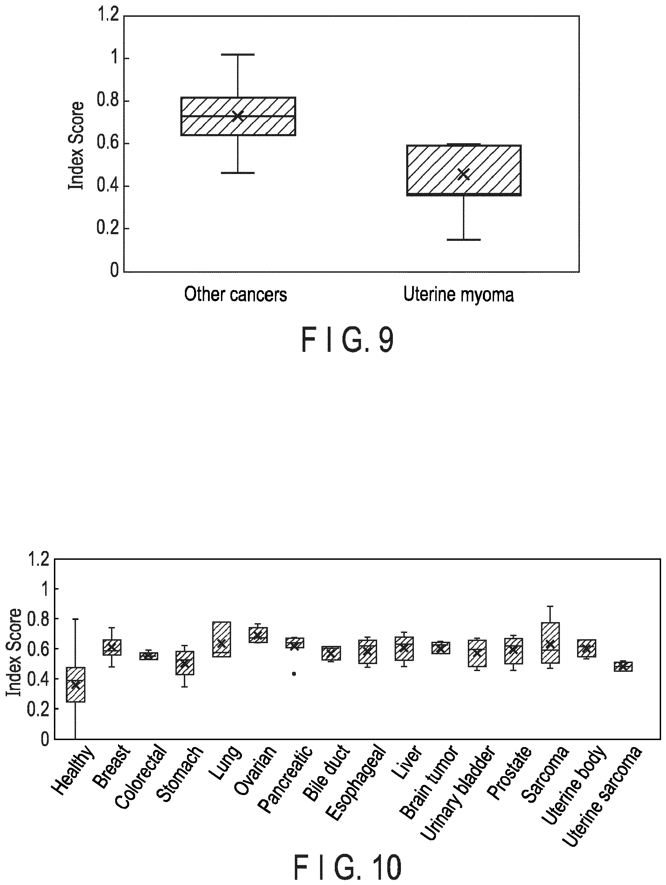

FIG. 9 is a box-and-whisker plot showing the results of classifying the corrected values of FIG. 10 into a target cancer patient group and a uterine sarcoma patient group. In the other cancer patient group, the corrected values were distributed at higher values than in the healthy subject group, and in the target cancer patient group, the corrected values were distributed at even higher values than in the uterine sarcoma patient group. The AUC dividing between the other cancer patient group and the patient group of four kinds of cancers was 0.95.

FIG. 10 shows the corrected values of hsa-miR-1301-3p in healthy subjects and various cancer patients in the case of using the primer set J. Regarding hsa-miR-1301-3p, the corrected values were distributed in the range of about 0.25 to about 0.50 in healthy subjects, while the corrected values were distributed in the range of high values of about 0.42 to about 0.80 in cancer patients. Furthermore, the corrected values were distributed in the range of higher values in the patients of the target cancers than in the uterine sarcoma patients. From these results, it was found that the abundance of hsa-miR-1301-3p in the blood serum was large in cancer patients, particularly patients of the target cancers.

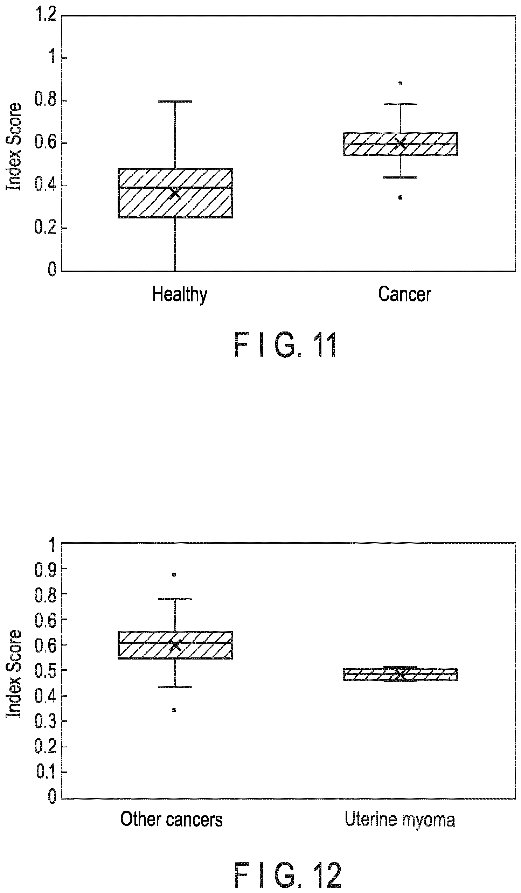

FIG. 11 is a box-and-whisker plot showing the results of classifying the corrected values of FIG. 10 into a healthy subject group and a cancer patient group. The cancer patient group was a group of combining the results of all the cancer patients other than healthy subjects, and the results were distributed at higher values than those of healthy subjects. The AUC dividing between the healthy subject group and the cancer patient group was 0.90.

FIG. 12 is a box-and-whisker plot showing the results of classifying the corrected values of FIG. 10 into a target cancer patient group and a uterine sarcoma patient group. In the other cancer patient group, the corrected values were distributed at higher values than in the healthy subject group, and in the target cancer patient group, the corrected values were distributed at even higher values than in the uterine sarcoma patient group. The AUC dividing between the uterine sarcoma group and the patient group of four kinds of cancers was 0.92.

Therefore, it was found that hsa-miR-1301-3p has high performance as a marker for distinguishing and detecting between a healthy subject and a cancer patient, or a marker for distinguishing and detecting between a target cancer and a healthy subject or a uterine sarcoma patient.

Furthermore, stability of the primer sets was evaluated. A known number of copies of hsa-miR-1301-3p was quantified by using a plurality of kinds of primer sets, and a calibration curve was prepared. Amplification was performed by using a real-time PCR apparatus, the fluorescence intensity was measured over time, and the time when it exceeds the threshold value was measured. Measurement was performed in duplicate, and the average value and CV of the time when it exceeds the threshold value were calculated. Quantification was repeated several times, and reproducibility of the formula of the calibration curve was evaluated. The results for the primer set A having excellent results are present in Table 5, and the results for the primer set J having superior results are present in Table 6. The calibration curve was prepared 6 times (run 1 to run 6) for the primer set A, and 5 times (run 1 to run 5) for the primer set J.

### Table 5

**Table 5. Primer set A**

| Number of miRNA copies | run1 | | run2 | | run3 | | run4 | | run5 | | run6 | |
|---|---|---|---|---|---|---|---|---|---|---|---|---|
| (10^n) | Ave. | CV% | Ave. | CV% | Ave. | CV% | Ave. | CV% | Ave. | CV% | Ave. | CV% |
| 6 | 16.2 | 2.52 | 19.3 | 0. 17 | 18 | 0.41 | 17.2 | 0. 79 | 18. 9 | 0.78 | 16.6 | 1.5 |
| 5 | 18.4 | 1.22 | 20.9 | 4.47 | 21.3 | 5.02 | 19.6 | 1. 14 | 20.8 | 1.81 | 18.8 | 1.79 |
| 4 | 20.5 | 1.2 | 24.6 | 0.71 | 23. 1 | 0.2 | 22.2 | 0.84 | 22. 6 | 4.33 | 20.9 | 0.57 |
| 3 | 22. 9 | 2.9 | 26.4 | 3. 03 | 25.8 | 2.77 | 25.4 | 0.61 | 25.5 | 4.27 | 23. 1 | 0.45 |
| 0 | 29. 1 | | UD | | 34.5 | | UD | 52.9/33.0 | | | UD | |
| Calibration curve | y=2.22x+29.5 | | y=2.49x+34.0 | | y=2.54x+33.4 | | y=2.72x+33.4 | | y=2.15x+31.6 | | y=2.19x+29.7 | |

### Table 6

**Table 6. Primer set J**

| Number of miRNA copies | run1 | | run2 | | run3 | | run4 | | run5 | |
|---|---|---|---|---|---|---|---|---|---|---|
| (5∗10^n) | Ave. | CV% | Ave. | CV% | Ave. | CV% | Ave. | CV% | Ave. | CV% |
| 5 | 19.9 | 0.18 | 19.9 | 0.34 | 20 | 0.41 | 20 | 0.06 | 19.9 | 1.11 |
| 4 | 23 | 1.18 | 22.5 | 0.13 | 22.8 | 0.48 | 22.8 | 0.08 | 22.3 | 0. 62 |
| 3 | 25.3 | 0.08 | 25.2 | 0.56 | 25. 7 | 0.09 | 25.8 | 0. 77 | 25. 1 | 1. 14 |
| 2 | 28.8 | 0. 64 | 28.4 | 1.91 | 28.7 | 2.77 | 28.4 | 0. 77 | 28.2 | 1.36 |
| 0 | UD | UD/54.5 | | | UD | | UD | | UD | |
| Calibration curve | y=2.90x+36.4 | | y=2.80x+35.3 | | y=2.82x+36.1 | | y=2.81x+36 | | y=2.75x+35.4 | |

As shown in Table 5, the gradient of the calibration curve for the primer set A was in the range of 2.15 to 2.72, and the fluctuation was small. Therefore, it was made clear that the primer set A is an excellent primer set capable of quantifying miRNA more stably.

As shown in Table 6, the gradient of the calibration curve for the primer set J was 2.75 to 2.90, and the fluctuation was even smaller compared to that of the primer set A. Furthermore, in run 1 through run 5, when the concentration of miRNA was 0 copies, rise of fluorescence was not observed, and it was made clear that non-specific amplification was unlikely to occur. Furthermore, in the primer set J, the fluctuation is small even when compared with the primer set A at the same concentration. For example, in the primer set A, the rise time at the time of 10⁶ copies shows fluctuations from run to run; however, regarding the primer set J, the rise time for 5×10⁵ copies shows smaller fluctuations from run to run.

From the above results, it was made clear that the primer set J is a superior primer set capable of quantifying miRNA more stably.

### Example 3. Electrochemical detection

A primer solution including the FIP primer and BIP Primer (48 µM each) used in Example 2 and an LB primer (24 µM) was prepared. 100 nL of the primer solution was spotted on a silicone flow channel gasket (flow channel width × height: 1 mm × 1 mm) using a microdispenser. A DNA chip substrate (glass (0.8 mm) / titanium (500 nm) / gold (2000 nm)) having a patterned electrode and the flow channel gasket were incorporated into a cassette, so that a chip was prepared.

Next, a LAMP amplification reaction solution having the composition shown in Table 7 was prepared.

**Table 7. Composition of LAMP amplification reaction liquid**

| Component | | Final concentration |
|---|---|---|
| | Tris-HCl(pH8.8) | 20 mM |
| | KCl | 60 mM |
| | MgSO₄ | 8 mM |
| | (NH₄)₂SO₄ | 10 mM |
| | Tween20 | 0.1 % |
| | dNTPs | 1.4 mM each |
| | Tin exo-DNA polymerase | 48 units(x3) |
| | Betaine | 0.8 M |
| | RuHex | 1 mM |
| Template | | 1 µL |
| Amount of reaction liquid | | 60 µL |

1 µL of the template after elongation used in Example 2 was added to the LAMP amplification reaction solution, and electrochemical measurement was performed under the conditions shown in Table 8.

**Table 8. Conditions for electrochemical measurement**

| Item | Details |
|---|---|
| Measurement method | Linear Sweep Voltammetry(LSV) |
| Sweep potential | 0.1 to -0.4 V |
| Sweep rate | 0.5 V/s |
| Temperature | 65°C |

When the LAMP amplification reaction was initiated, the reduction current value of ruthenium hexaamine (RuHex) began to increase. It was made clear that the time for the current to increase is faster as the abundance of the elongation product is larger, and quantitative detection can be achieved by using the present chip.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. An analytical method for determining the presence or absence of contraction of cancer in a subject, the method comprising quantifying hsa-miR-1301-3p in a sample originating from the subject.

2. An analytical method for assisting in determining the presence or absence of contraction of cancer in a subject, the method comprising quantifying hsa-miR-1301-3p in a sample originating from the subject.

3. The method according to claim 1 or 2, wherein the method further comprises determining that when an abundance of hsa-miR-1301-3p in the subject is larger than the abundance of hsa-miR-1301-3p in a control, the subject has contracted cancer.

4. The method according to any one of claims 1 to 3, wherein the determining the presence or absence of contraction of cancer includes determining the prognosis of cancer in the subject or the presence or absence of recurrence of cancer in the subject.

5. The method according to any one of claims 1 to 4, wherein the cancer includes at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, uterine body cancer, and uterine sarcoma.

6. The method according to any one of claims 1 to 4, wherein the quantification is carried out using a LAMP method, and
the cancer is at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, and uterine body cancer.

7. The method according to claim 6, wherein with regard to the determining, the presence or absence of contraction of at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, or uterine body cancer is determined by distinguishing from another cancer.

8. The method according to claim 7, wherein the other cancer includes uterine sarcoma.

9. The method according to any one of claims 6 to 8, wherein the cancer is prostate cancer,
the method further comprises quantifying hsa-miR-92a-3p in the sample, and
the determining is carried out by using the quantification results for hsa-miR-1301-3p and the quantification results for hsa-miR-92a-3p.

10. The method according to any one of claims 6 to 8, wherein the cancer is pancreatic cancer,
the method further comprises quantifying hsa-miR-122-5p in the sample, and
the determining is carried out by using the quantification results for hsa-miR-1301-3p and the quantification results for hsa-miR-122-5p.

11. The method according to any one of claims 1 to 10, wherein the method further comprises adding a miRNA that is not present in the subject to the sample and quantifying the miRNA, and the results of the quantifying for hsa-miR-1301-3p are corrected by comparing with the results of the quantifying for the miRNA that is not present in the subject.

12. The method according to any one of claims 1 to 11, wherein the sample is blood serum.

13. The method according to any one of claims 1 to 12, wherein the quantifying is carried out by using at least one nucleic acid selected from the group consisting of a reverse transcription primer for reverse transcribing hsa-miR-1301-3p, an elongation primer for elongating hsa-miR-1301-3p, an amplification primer set for amplifying hsa-miR-1301-3p, and a nucleic acid probe for detecting hsa-miR-1301-3p.

14. The method according to claim 13, wherein the reverse transcription primer contains SEQ ID NO: 4, the elongation primer contains SEQ ID NO: 5, and the amplification primer set includes an FIP primer set forth in SEQ ID NO: 6, a BIP primer set forth in SEQ ID NO: 7, and an LB primer set forth in SEQ ID NO: 8,
the reverse transcription primer contains SEQ ID NO: 9, the elongation primer contains SEQ ID NO: 10, and the amplification primer set includes an FIP primer set forth in SEQ ID NO: 11, a BIP primer set forth in SEQ ID NO: 12, and the LB primer set forth in SEQ ID NO: 8,
the reverse transcription primer contains SEQ ID NO: 13, the elongation primer contains SEQ ID NO: 14, and the amplification primer set includes an FIP primer set forth in SEQ ID NO: 15, a BIP primer set forth in SEQ ID NO: 16, and the LB primer set forth in SEQ ID NO: 8,
the reverse transcription primer contains SEQ ID NO: 17, the elongation primer contains SEQ ID NO: 18, and the amplification primer set includes an FIP primer set forth in SEQ ID NO: 19, a BIP primer set forth in SEQ ID NO: 20, and the LB primer set forth in SEQ ID NO: 8, or
the reverse transcription primer contains SEQ ID NO: 45, the elongation primer contains SEQ ID NO: 46, and the amplification primer set includes an FIP primer set forth in SEQ ID NO: 47, a BIP primer set forth in SEQ ID NO: 48, and the LB primer set forth in SEQ ID NO: 8, and
a base sequence of each of the primers also includes a base sequence set forth in the corresponding SEQ ID NO and containing deletion, substitution, addition, or insertion of one or two bases.

15. A kit for detecting cancer, the kit comprising at least one nucleic acid selected from the group consisting of a reverse transcription primer for reverse transcribing hsa-miR-1301-3p, an elongation primer for elongating hsa-miR-1301-3p, an amplification primer set for amplifying hsa-miR-1301-3p, and a nucleic acid probe for detecting hsa-miR-1301-3p.

16. The kit according to claim 15, wherein
the reverse transcription primer contains SEQ ID NO: 4, the elongation primer contains SEQ ID NO: 5, and the amplification primer set includes an FIP primer set forth in SEQ ID NO: 6, a BIP primer set forth in SEQ ID NO: 7, and an LB primer set forth in SEQ ID NO: 8,
the reverse transcription primer contains SEQ ID NO: 9, the elongation primer contains SEQ ID NO: 10, and the amplification primer set includes an FIP primer set forth in SEQ ID NO: 11, a BIP primer set forth in SEQ ID NO: 12, and the LB primer set forth in SEQ ID NO: 8,
the reverse transcription primer contains SEQ ID NO: 13, the elongation primer contains SEQ ID NO: 14, and the amplification primer set includes an FIP primer set forth in SEQ ID NO: 15, a BIP primer set forth in SEQ ID NO: 16, and the LB primer set forth in SEQ ID NO: 8,
the reverse transcription primer contains SEQ ID NO: 17, the elongation primer contains SEQ ID NO: 18, and the amplification primer set includes an FIP primer set forth in SEQ ID NO: 19, a BIP primer set forth in SEQ ID NO: 20, and the LB primer set forth in SEQ ID NO: 8, or
the reverse transcription primer contains SEQ ID NO: 45, the elongation primer contains SEQ ID NO: 46, and the amplification primer set includes an FIP primer set forth in SEQ ID NO: 47, a BIP primer set forth in SEQ ID NO: 48, and the LB primer set forth in SEQ ID NO: 8, and
a base sequence of each of the primers also includes a base sequence set forth in the corresponding SEQ ID NO and containing deletion, substitution, addition, or insertion of one or two bases.

17. The kit according to claim 15 or 16, wherein the cancer includes at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, uterine body cancer, and uterine sarcoma.

18. The kit according to claim 17, wherein the cancer includes at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, and uterine body cancer.

19. The kit according to claim 18, wherein the kit further comprises at least one nucleic acid selected from the group consisting of a reverse transcription primer, an elongation primer, an amplification primer set, and a nucleic acid probe for hsa-miR-92a-3p.

20. The kit according to claim 18, wherein the kit further comprises at least one nucleic acid selected from the group consisting of a reverse transcription primer, an elongation primer, an amplification primer set, and a nucleic acid probe for hsa-miR-122-5p.

21. The kit according to any one of claims 15 to 20, wherein the kit further comprises:
cel-miR-39-3p; and
at least one nucleic acid selected from the group consisting of a reverse transcription primer, an elongation primer, an amplification primer set, and a nucleic acid probe for cel-miR-39-3p.

22. A primer set for detecting cancer, wherein the primer set comprising:
the reverse transcription primer contains SEQ ID NO: 4, the elongation primer contains SEQ ID NO: 5, and the amplification primer set includes an FIP primer set forth in SEQ ID NO: 6, a BIP primer set forth in SEQ ID NO: 7, and an LB primer set forth in SEQ ID NO: 8,
the reverse transcription primer contains SEQ ID NO: 9, the elongation primer contains SEQ ID NO: 10, and the amplification primer set includes an FIP primer set forth in SEQ ID NO: 11, a BIP primer set forth in SEQ ID NO: 12, and the LB primer set forth in SEQ ID NO: 8,
the reverse transcription primer contains SEQ ID NO: 13, the elongation primer contains SEQ ID NO: 14, and the amplification primer set includes an FIP primer set forth in SEQ ID NO: 15, a BIP primer set forth in SEQ ID NO: 16, and the LB primer set forth in SEQ ID NO: 8,
the reverse transcription primer contains SEQ ID NO: 17, the elongation primer contains SEQ ID NO: 18, and the amplification primer set includes an FIP primer set forth in SEQ ID NO: 19, a BIP primer set forth in SEQ ID NO: 20, and the LB primer set forth in SEQ ID NO: 8, or
the reverse transcription primer contains SEQ ID NO: 45, the elongation primer contains SEQ ID NO: 46, and the amplification primer set includes an FIP primer set forth in SEQ ID NO: 47, a BIP primer set forth in SEQ ID NO: 48, and the LB primer set forth in SEQ ID NO: 8, and
a base sequence of each of the primers also includes a base sequence set forth in the corresponding SEQ ID NO and containing deletion, substitution, addition, or insertion of one or two bases.

23. The primer set according to claim 22, wherein the cancer includes at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, and uterine body cancer.

24. A detection device for detecting cancer, the detection device comprising an amplification detection element that amplifies hsa-miR-1301-3p and detects an amplification product,
wherein the amplification detection element includes a substrate and one or more detection regions provided on one surface of the substrate.

25. The detection device according to claim 24, wherein the cancer includes at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, uterine body cancer, and uterine sarcoma.

26. The detection device according to claim 24 or 25, wherein the detection device comprises a nucleic acid probe immobilized in the vicinity of the detection region, the nucleic acid probe being hybridized with the amplification product.

27. The detection device according to any one of claims 24 to 26, wherein the detection device further comprises a reverse transcription primer for reverse transcribing hsa-miR-1301-3p, an elongation primer for elongating hsa-miR-1301-3p, and/or an amplification primer set for amplifying hsa-miR-1301-3p, these primers being detachably immobilized in the vicinity of the detection region.

28. The detection device according to claim 27, wherein
the reverse transcription primer contains SEQ ID NO: 4, the elongation primer contains SEQ ID NO: 5, and the amplification primer set includes an FIP primer set forth in SEQ ID NO: 6, a BIP primer set forth in SEQ ID NO: 7, and an LB primer set forth in SEQ ID NO: 8,
the reverse transcription primer contains SEQ ID NO: 9, the elongation primer contains SEQ ID NO: 10, and the amplification primer set includes an FIP primer set forth in SEQ ID NO: 11, a BIP primer set forth in SEQ ID NO: 12, and the LB primer set forth in SEQ ID NO: 8,
the reverse transcription primer contains SEQ ID NO: 13, the elongation primer contains SEQ ID NO: 14, and the amplification primer set includes an FIP primer set forth in SEQ ID NO: 15, a BIP primer set forth in SEQ ID NO: 16, and the LB primer set forth in SEQ ID NO: 8,
the reverse transcription primer contains SEQ ID NO: 17, the elongation primer contains SEQ ID NO: 18, and the amplification primer set includes an FIP primer set forth in SEQ ID NO: 19, a BIP primer set forth in SEQ ID NO: 20, and the LB primer set forth in SEQ ID NO: 8, or
the reverse transcription primer contains SEQ ID NO: 45, the elongation primer contains SEQ ID NO: 46, and the amplification primer set includes an FIP primer set forth in SEQ ID NO: 47, a BIP primer set forth in SEQ ID NO: 48, and the LB primer set forth in SEQ ID NO: 8, and
a base sequence of each of the primers also includes a base sequence set forth in the corresponding sequence number and containing deletion, substitution, addition, or insertion of one or two bases.

29. The detection device according to claim 28, wherein the cancer includes at least one selected from breast cancer, colorectal cancer, stomach cancer, lung cancer, ovarian cancer, pancreatic cancer, bile duct cancer, esophageal cancer, liver cancer, brain tumor, urinary bladder cancer, prostate cancer, sarcoma, and uterine body cancer.

30. The device according to any one of claims 24 to 29, wherein the device further comprises a calculation element that calculates a quantitative value of hsa-miR-1301-3p from the results of the detecting.


SEQUENCE LISTING

<110>  Kabushiki Kaisha Toshiba

<120>  Analytical method and Kit

<130>  20S0362PCT

<160>  54

<170>  PatentIn version 3.5

<210>  1
<211>  24
<212>  RNA
<213>  human

<400>  1
uugcagcugc cugggaguga cuuc                                              24

<210>  2
<211>  22
<212>  RNA
<213>  human

<400>  2
uauugcacuu gucccggccu gu                                                22

<210>  3
<211>  22
<212>  RNA
<213>  human

<400>  3
uggaguguga caaugguguu ug                                                22

<210>  4
<211>  29
<212>  DNA
<213>  artificial

<220>
<223>  RT primer

<400>  4
aggatcgtac gagaccgaag gaagtcact                                         29

<210>  5
<211>  77
<212>  DNA
<213>  artificial

<220>
<223>  EL primer

<400>  5
gagtcgtcgc cagtacctcg gttggagctg aattttgcgt ggaccgacgg cttgatgcag      60

ggttgcagct gcctggg                                                      77

<210>  6
<211>  42
<212>  DNA
<213>  artificial

<220>
<223>  FIP primer

<400>  6
acgcaaaatt cagctccaac cgttgagtcg tcgccagtac ct                          42


<210>  7
<211>  39
<212>  DNA
<213>  artificial

<220>
<223>  BIP primer

<400>  7
ggaccgacgg cttgatgcaa ggatcgtacg agaccgaag                             39


<210>  8
<211>  17
<212>  DNA
<213>  artificial

<220>
<223>  LB primer

<400>  8
tgcctgggag tgacttc                                                     17


<210>  9
<211>  28
<212>  DNA
<213>  artificial

<220>
<223>  RT primer

<400>  9
ctgcaacgtt gaacatgcgg aagtcact                                         28


<210>  10
<211>  82
<212>  DNA
<213>  artificial

<220>
<223>  EL primer

<400>  10
cttcgtgcag atggctatgc ggctagaata cggcacgact gctgatgtgt ctgatagcgg      60

cacggggttg cagctgcctg gg                                               82

<210>  11
<211>  42
<212>  DNA
<213>  artificial

<220>
<223>  FIP primer

<400>  11
gcagtcgtgc cgtattctag cccttcgtgc agatggctat gc                    42


<210>  12
<211>  41
<212>  DNA
<213>  artificial

<220>
<223>  BIP primer

<400>  12
tgatgtgtct gatagcggca cgctgcaacg ttgaacatgc g                     41


<210>  13
<211>  29
<212>  DNA
<213>  artificial

<220>
<223>  RT primer

<400>  13
tctgctctgg ttgctctcat gaagtcact                                   29


<210>  14
<211>  80
<212>  DNA
<213>  artificial

<220>
<223>  EL primer

<400>  14
atctgctgca ccactgaaag ctcggtagac gcatagtcgc gagtcgggaa ccctgtgggt    60

ttgggttgca gctgcctggg                                             80


<210>  15
<211>  41
<212>  DNA
<213>  artificial

<220>
<223>  FIP primer

<400>  15
cgcgactatg cgtctaccga gatctgctgc accactgaaa g                     41

<210>  16
<211>  41
<212>  DNA
<213>  artificial

<220>
<223>  BIP primer

<400>  16
agtcgggaac cctgtgggtt ttctgctctg gttgctctca t                41


<210>  17
<211>  29
<212>  DNA
<213>  artificial

<220>
<223>  RT primer

<400>  17
ggcgccgaaa caatattcct gaagtcact                29


<210>  18
<211>  78
<212>  DNA
<213>  artificial

<220>
<223>  ET primer

<400>  18
gatctagaag gccgccagtc gttcagccta cggccgttgt catccgtagc aggacgctca        60

gggttgcagc tgcctggg                78


<210>  19
<211>  38
<212>  DNA
<213>  artificial

<220>
<223>  FIP primer

<400>  19
aacggccgta ggctgaacgg atctagaagg ccgccagt                38


<210>  20
<211>  42
<212>  DNA
<213>  artificial

<220>
<223>  BIP primer

<400>  20
gtcatccgta gcaggacgct caggcgccga aacaatattc ct                42


<210>  21

<211>  26
<212>  DNA
<213>  artificial

<220>
<223>  RT primer

<400>  21
ggaggcgaca cgagttctac aggccg                                          26


<210>  22
<211>  79
<212>  DNA
<213>  artificial

<220>
<223>  EL primer

<400>  22
ggctgtgcag agataggtgg tacgaagcag ccttctcggc tcggctatct acgcgttaag     60

cggggtattg cacttgtcc                                                  79


<210>  23
<211>  39
<212>  DNA
<213>  artificial

<220>
<223>  FIP primer

<400>  23
gccgagaagg ctgcttcgta ggctgtgcag agataggtg                            39


<210>  24
<211>  40
<212>  DNA
<213>  artificial

<220>
<223>  BIP primer

<400>  24
tcggctatct acgcgttaag cgggaggcga cacgagttct                           40


<210>  25
<211>  16
<212>  DNA
<213>  artificial

<220>
<223>  LB primer

<400>  25
acttgtcccg gcctgt                                                     16


<210>  26
<211>  17

<212>  DNA
<213>  artificial

<220>
<223>  LB primer

<400>  26
ttgtcccggc ctgtaga                                                    17


<210>  27
<211>  28
<212>  DNA
<213>  artificial

<220>
<223>  RT primer

<400>  27
ggcgccgaaa caatattcct acaggccg                                        28


<210>  28
<211>  77
<212>  DNA
<213>  artificial

<220>
<223>  EL primer

<400>  28
gatctagaag gccgccagtc gttcagccta cggccgttgt catccgtagc aggacgctca     60

gggtattgca cttgtcc                                                    77


<210>  29
<211>  38
<212>  DNA
<213>  artificial

<220>
<223>  FIP primer

<400>  29
aacggccgta ggctgaacgg atctagaagg ccgccagt                             38


<210>  30
<211>  42
<212>  DNA
<213>  artificial

<220>
<223>  BIP primer

<400>  30
gtcatccgta gcaggacgct caggcgccga aacaatattc ct                        42


<210>  31
<211>  27
<212>  DNA

<213>  artificial

<220>
<223>  RT primer

<400>  31
ctgcaacgtt gaacatgcga caggccg                                          27


<210>  32
<211>  80
<212>  DNA
<213>  artificial

<220>
<223>  EL primer

<400>  32
ttcgtgcaga tggctatgcg gctagaatac ggcacgactg ctgatgtgtc tgatagcggc      60

acggggtatt gcacttgtcc                                                  80


<210>  33
<211>  42
<212>  DNA
<213>  artificial

<220>
<223>  FIP primer

<400>  33
gcagtcgtgc cgtattctag cccttcgtgc agatggctat gc                         42


<210>  34
<211>  41
<212>  DNA
<213>  artificial

<220>
<223>  BIP primer

<400>  34
tgatgtgtct gatagcggca cgctgcaacg ttgaacatgc g                          41


<210>  35
<211>  28
<212>  DNA
<213>  artificial

<220>
<223>  RT primer

<400>  35
cggaacggca taaaaagccc aaacacca                                         28


<210>  36
<211>  77
<212>  DNA
<213>  artificial

<220>
<223>  EL primer

<400>  36
gaatgtgacc acgcggatac agactttcga tccacgctgg gaccgaggcc agactctacc      60

tgggtggagt gtgacaa                                                     77


<210>  37
<211>  40
<212>  DNA
<213>  artificial

<220>
<223>  FIP primer

<400>  37
ccagcgtgga tcgaaagtct gtgaatgtga ccacgcggat                            40


<210>  38
<211>  40
<212>  DNA
<213>  artificial

<220>
<223>  BIP primer

<400>  38
gaccgaggcc agactctacc tcggaacggc ataaaaagcc                            40


<210>  39
<211>  18
<212>  DNA
<213>  artificial

<220>
<223>  LB primer

<400>  39
gtgtgacaat ggtgtttg                                                    18


<210>  40
<211>  18
<212>  DNA
<213>  artificial

<220>
<223>  LB primer

<400>  40
tgacaatggt gtttgggc                                                    18


<210>  41
<211>  29
<212>  DNA
<213>  artificial

<220>
<223>  RT primer

<400>  41
ggcgccgaaa caatattcct caaacacca                                          29


<210>  42
<211>  76
<212>  DNA
<213>  artificial

<220>
<223>  EL primer

<400>  42
gatctagaag gccgccagtc gttcagccta cggccgttgt catccgtagc aggacgctca        60

gggtggagtg tgacaa                                                        76


<210>  43
<211>  38
<212>  DNA
<213>  artificial

<220>
<223>  FIP primer

<400>  43
aacggccgta ggctgaacgg atctagaagg ccgccagt                                38


<210>  44
<211>  42
<212>  DNA
<213>  artificial

<220>
<223>  BIP primer

<400>  44
gtcatccgta gcaggacgct caggcgccga aacaatattc ct                           42

<210>  45
<211>  29
<212>  DNA
<213>  artificial

<220>
<223>  RT Primer

<400>  45
atgagaggac atacctccgc gaagtcact                                          29


<210>  46
<211>  79
<212>  DNA
<213>  artificial

<220>
<223>  EL Primer

<400>  46
tcagtcgggg acaatccggt cactagagtc ccggtcggtt cggcgaatag ggctcccagt      60

agggttgcag ctgcctggg                                                   79


<210>  47
<211>  40
<212>  DNA
<213>  artificial

<220>
<223>  FIP Primer

<400>  47
aaccgaccgg gactctagtg actcagtcgg ggacaatccg                            40


<210>  48
<211>  41
<212>  DNA
<213>  artificial

<220>
<223>  BIP Primer

<400>  48
cggcgaatag ggctcccagt aatgagagga catacctccg c                          41


<210>  49
<211>  22
<212>  RNA
<213>  C. elegans

<400>  49
ucaccgggug uaaaucagcu ug                                               22


<210>  50
<211>  48
<212>  DNA
<213>  artificial

<220>
<223>  RT Primer

<400>  50
tcaggttcag atcatcccca gggtctgcag acgtttctcc aagctgat                   48


<210>  51
<211>  80
<212>  DNA
<213>  artificial

<220>
<223>  EL Primer

<400>  51
atacggtcat tgctgctcct acgaggcaga acaatgccct cgggtagttg aggcaccatg      60

acgtgggtca ccgggtgtaa                                                    80

<210>  52
<211>  46
<212>  DNA
<213>  artificial

<220>
<223>  FIP Primer

<400>  52
gagggcattg ttctgcctcg tattttaata cggtcattgc tgctcc                      46

<210>  53
<211>  42
<212>  DNA
<213>  artificial

<220>
<223>  BIP Primer

<400>  53
ggtagttgag gcaccatgac gttcaggttc agatcatccc ca                          42

<210>  54
<211>  16
<212>  DNA
<213>  artificial

<220>
<223>  LB Primer

<400>  54
ggtgtaaatc agcttg                                                       16